(19) Europäisches Patentamt
European Patent Office
Office européen des brevets

(11) Numéro de publication: **0 352 164 A1**

(12) # DEMANDE DE BREVET EUROPEEN

(21) Numéro de dépôt: **89401957.9**

(22) Date de dépôt: **07.07.89**

(51) Int. Cl.$^5$: **C 07 B 35/06**
C 07 C 1/26, C 07 C 41/24,
C 07 C 67/30, C 07 F 15/00

(30) Priorité: **20.07.88 FR 8809790**

(43) Date de publication de la demande:
**24.01.90 Bulletin 90/04**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Huser, Marc**
**36, rue du Fossé Rietberg**
**F-67100 Strasbourg (FR)**

**Osborn, John**
**10, rue Daniel Hirtz**
**F-67000 Strasbourg (FR)**

(74) Mandataire: **Vignally, Noel et al**
**Rhône-Poulenc Chimie Service Brevets Chimie 25 Quai Paul Doumer**
**F-92408 Courbevoie Cédex (FR)**

(54) **Procédé d'hydrogénolyse.**

(57) La présente invention concerne un procédé d'hydrogénolyse mettant en oeuvre un nouveau catalyseur constitue d'un complexe palladium-phosphine, celle-ci présentant un pKa supérieur ou égal à 6, un dérivé aromatique chloré et de l'hydrogène.



Bundesdruckerei Berlin

## Description

# PROCEDE D'HYDROGENOLYSE

La présente invention concerne un procédé d'hydrogénolyse. Elle concerne plus particulièrement l'hydrogénolyse de dérivés aromatiques chlorés en présence d'un catalyseur à base de palladium.

Il est intéressant de procéder à l'hydrogénolyse de composés aromatiques chlorés lorsque, par exemple, il est nécessaire de conserver un ou plusieurs substituants qui n'ont pu être apportés en une position précise du noyau aromatique que grâce à l'atome de chlore.

Nous ne connaissons pas à ce jour de documents décrivant l'hydrogénolyse de composés chlorés au moyen de catalyseurs en phase homogène.

La présente invention a permis d'atteindre cet objectif, c'est-à-dire qu'il est maintenant possible d'hydrogénolyser en phase liquide homogène un dérivé aromatique chloré en présence d'un catalyseur à base de palladium.

Le catalyseur à base de palladium est précisément choisi parmi les complexes du palladium et d'une phosphine. Cette phosphine doit présenter un pKa supérieur à 6, tel que défini par WmA HENDERSON, Jr ; C.A. STREULI dans le journal of American Chemical Society, 82, 1960, 5791.

Parmi les phosphines présentant un pKa supérieur à 6, on peut citer non limitativement :
- la tricyclohexylphosphine
- la tribenzylphosphine
- la triisopropylphosphine
- la triisobutylphosphine
- la phényldicyclohexyl phosphine
- la triethylphosphine
- la tributylphosphine
- la tripropylphosphine

Parmi l'ensemble de ces phosphines, on préfère utilise celles qui ont la double caractéristique pKa supérieur à 6 et angle de cône tel que défini par C.A. TOLMAN, dans le Journal of American Chemical Society, 92, 1970, 2956 compris entre 160 et 180°.

Rentrent notamment dans ce domaine les phosphines suivantes :
- la tricyclohexylphosphine
- la dicyclohexylphénylphosphine
- la triisopropylphosphine
- la tribenzylphosphine

On préfère utiliser la tricyclohexylphosphine.

Le complexe de la présente invention répond à la formule (I) suivante :

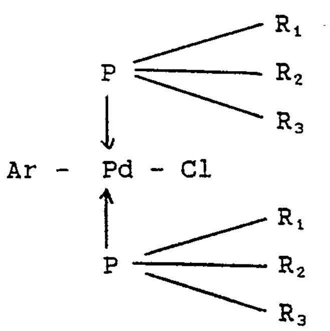

(I)

dans laquelle
- $R_1$, $R_2$, $R_3$ représentent chacun des groupes identiques ou différents choisis parmi les radicaux cyclohexyle, benzyle, isopropyle, l'un des groupes $R_1$, $R_2$ ou $R_3$ pouvant être remplacé par un groupe phényl lorsque les deux autres représentent un groupe cyclohexyle,
- Ar représente un radical aromatique mono, polycyclique ou hétérocyclique éventuellement substitué.

Le complexe de formule (I) précédemment décrit est notamment utilisé pour catalyser les réactions d'hydrogénolyse selon le mécanisme réactionnel suivant :

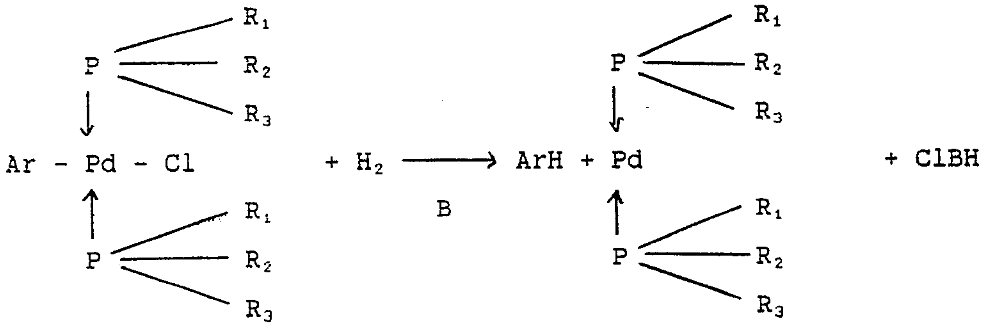

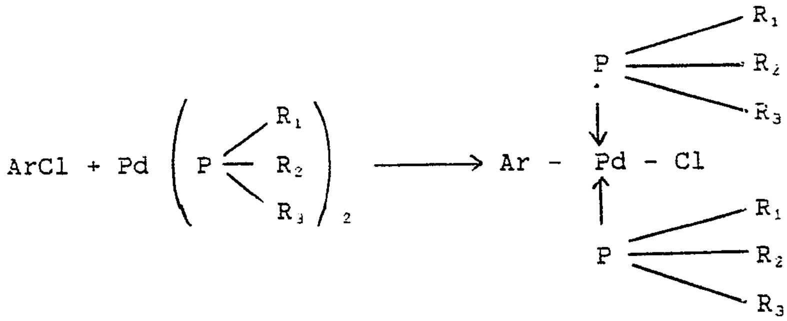

qui d'une manière simplifiée peut être résumée par l'équation suivante :

$$ArCl + H_2 \xrightarrow[B]{\text{Catalyseur à base de Pd}} ArH + Cl\ B\ H$$

Dans les équations précédentes, le terme "R" signifie soit un groupe cyclohexyl, soit un groupe phényl, soit un groupe benzyl, soit un groupe isopropyl. Le phosphore peut être ligandé à 3 groupes équivalents comme dans la tricyclohexyl phosphine ou par des groupes différents comme dans la dicyclohexylphényl phosphine.

Le dérivé aromatique chloré (ArCl) peut être mono, polycyclique ou hétérocyclique. Il peut être éventuellement substitué par un groupe alkoxy, alkyle, alkylcarbonyle, cycloalkyle, cycloalkoxy, halogéno, halogénoalkye, halogénoalkoxy, aryle, aryloxy, halogénoaryle, halogénoaryloxy, alkylaryle, aralkyle, alkylcarbonyloxy, cycloalkylcarbonyloxy, arylcarbonyloxy, aryloxycarbonyle.

Les chaînes alkyle des divers groupes alkyle ou alkoxy contiennent de préférence 1 à 6 atomes de carbone, les groupes aryle contiennent de préférence 6 à 18 atomes de carbone.

On préfère utiliser les dérivés aromatiques monocycliques non substitués ou substitués par un groupe alkoxy, contenant 1 à 6 atomes de carbone, alkyle contenant 1 à 6 atoms de carbone, chloro, fluoro, alkyl carbonyle dont la chaîne alkyle contient 1 à 6 atomes de carbone.

Parmi les dérivés aromatiques chlorés utilisables dans le procédé de l'invention, on peut citer à titre illustratif :
- le chlorobenzène
- le dichlorobenzene
- le chlorofluorobenzene
- les chlorotoluènes
- les chloroanisoles
- les chloronaphthalenes
- les méthyl, éthyl, propylchlorobenzoates
- la méthylchlorophénylcétone
- les chlorobiphényles
- le chloroindole
- le chlorothiophène

Une base (B) est nécessaire pour neutraliser l'acide chlorhydrique formé au cours de la réaction d'hydrogénolyse. Cette base peut être constituée de la phosphine elle-même ou d'une base différente. Si cette base est différente, elle a de préférence un pKa supérieur à celui de la phosphine de manière à ce que cette dernière ne joue pas inutilement le rôle de base neutralisante.

Elle doit de préférence être soluble dans le milieu réactionnel. On préfère utiliser les trialkylamines et par exemple la triéthylamine, la triisopropylamine, la tri-n butylamine. Les bases minérales peuvent aussi être utilisées telles que le carbonate de sodium mais n'apportent pas d'avantage particulier.

Le solvant utilisé pour la mise en oeuvre de l'invention est choisi parmi les solvants aromatiques hydrocarbonés tels que :
- le toluène
- les xylènes

les éthers, tels que :
- le dioxane

les alcools, tels que :
- l'éthanol
- l'isopropanol

les cétones, telles que :
- la méthylisobutylcétone

les nitriles, tels que :
- le benzonitrile

les amides, tels que :
- le diméthylformamide

Les réactifs tels que le dérivé chloro aromatique ou la base peuvent servir de milieu réactionnel.

Le complexe de formule I peut être utilisé tel quel comme catalyseur.

Le complexe de formule I peut être aussi formé "in situ" par au moins trois méthodes de mise en oeuvre.

Selon une première méthode de mise en oeuvre du procédé de l'invention, on met en contact un composé de formule (II) suivante :

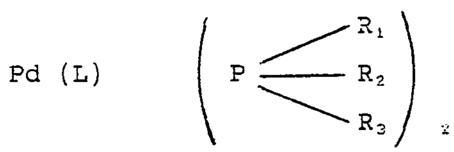

( II )

dans laquelle
- le motif L représente un groupe labile en présence de ArCl.
- les groupes $R_1$, $R_2$, $R_3$ ont la même signification que dans la formule (I) avec un dérivé halogéno aromatique de formule ArCl et l'hydrogène dans un solvant.

Selon une deuxième méthode de mise en oeuvre du procédé de l'invention on met en présence un complexe de palladium à l'état d'oxydation zéro tel que :
Pd $(L)_3$, au moins deux équivalents de phosphine répondant à la formule

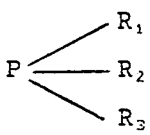

avec le dérivé chloroaromatique de formule ArCl et de l'hydrogène.

Selon une troisième méthode de mise en oeuvre du procédé de l'invention, on met en présence un sel de palladium à l'état d'oxydation II choisi par exemple parmi le dichlorure, le dibromure, le diiodure de palladium, le diacétate de palladium, le nitrate de palladium, le sulfate de palladium, l'oxyde de palladiuum avec le dérivé chloroaromatique, et au moins deux équivalents de phosphine de formule

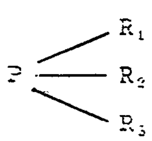

et en présence d'hydrogène.

Au sens de la présente invention, on entend par groupe labile (L) tout groupe qui en présence de ArCl peut être facilement échangeable.

Parmi ces groupes, on peut citer non limitativement :
- la dibenzylidène acétone (DBA)
- les groupes alkylène et de préférence l'éthylène

Lorsque l'on part d'un complexe du palladium ne contenant pas de phosphine (deuxième ou troisième méthode de mise en oeuvre), on préfère utiliser au moins 2 mol de phosphine par atome gramme de palladium et de préférence entre 2 et 10000 mol et tout particulièrement entre 2 et 5.

On préfère utiliser une quantité de solvant telle que la concentration en complexe ou en sel de palladium dans le milieu soit comprise entre $10^{-5}$ et 100 mmol par litre.

La concentration minimale en base doit correspondre à la stoéchiométrie de la réaction. Elle peut être utilisée en quantité nettement supérieure et même être utilisée comme solvant. Il est souhaitable qu'en fin de réaction, on n'est pas épuisé l'amine.

La concentration du dérivé aromatique chloré peut varier dans de larges limites puisqu'il peut être utilisé comme solvant. Il est dans ce cas facilement recyclé.

La température réactionnelle est avantageusement comprise entre 50 et 250°C et de préférence entre 100 et 200°C.

La pression partielle d'hydrogène est notamment comprise entre 1 et 300 bar et de préférence entre 10 et 100 bar.

La présente invention sera plus complètement décrite à l'acide des exemples suivants qui ne doivent pas être considérés comme limitatifs de l'invention.

Dans les exemples suivants les abréviations suivants signifient:

$PCy_3$ = tricyclohexyl phosphine

$$TT = \text{taux de transformation} \quad \frac{\text{quantité de dérivé aromatique halogéné transformée}}{\text{quantité de dérivé aromatique halogéné introduite}} \times 100$$

$$RT = \text{rendement sur produit transformé} \quad \frac{\text{quantité de produit désiré formé (mol)}}{\text{quantité de produit transformé (mol)}} \times 100$$

EXEMPLES 1 et 4

INFLUENCE DE LA NATURE DE LA PHOSPHINE

Dans un réacteur en hastelloy HB2®, on introduit :

. 1 matg de diacétate de palladium
. 5 mmol de $PL_3$ en faisant varier L
. 50 mmol de $RC_6H_4Cl$
. 110 mmol de $NEt_3$
. qsp 30 ml de toluène

On maintient le réacteur sous une pression d'hydrogène de 15 bar, à une température de 180°C pendant 4 heures.

| Ex | R | Phosphine $PL_3$ | TT | RT(p-$RC_6H_5$) |
|---|---|---|---|---|
| 1 | H | $PCy_3$ | 29 | 100 |
| 2 | H | $PBz_3$ | 41 | 85 |
| 3 | COOEt | $PBz_3$ | 48 | 88 |
| 4 | H | $PEt_3$ | 10 | 100 |

Un essai comparatif a été réalisé en remplaçant la phosphine $PL_3$ par une phosphine ayant un pKa inférieur à 6, la triphénylphosphine. Le milieu est devenu hétérogène et nous avons dû stopper l'essai.

EXEMPLES 5 ET 6

INFLUENCE DE LA NATURE D'UN SUBSTITUANT SUR p-$RC_6H_4Cl$

Dans le même réacteur que précédemment, on introduit :

. 1 matg de Pd $(OAc)_2$
. 5 mmol de $PCy_3$
. 50 mmol de p-$RC_6H_4Cl$

. 110 mmol de $NEt_3$
. qsp 30 ml de toluène

On maintient le réacteur sous une pression d'hydrogène de 15 bar, à une température de 180° C pendant 4 heures.

| Ex | R | TT | RT(p-$RC_6H_5$) |
|---|---|---|---|
| 5 | OMe | 22 | 82 |
| 1 | H | 29 | 100 |
| 6 | COOEt | 86 | 100 |

EXEMPLE 7

INFLUENCE DE LA CONCENTRATION DE LA BASE

On reprend l'exemple 1 en utilisant 5 mmol de $PCy_3$ et 179 mmol de $NEt_3$.

| Ex | [$NEt_3$] | TT | RT($C_6H_6$) |
|---|---|---|---|
| 1 | 100 | 29 | 100 |
| 7 | 179 | 52 | 98 |

Un essai comparatif a été réalisé avec une amine (la pyridine) ayant un pKa inférieur à celui de la phosphine, le taux de transformation du chlorobenzène est nul.

**Revendications**

1. Procédé d'hydrogénolyse de composés aromatiques halogénés caractérisé en ce que l'on met en présence un dérivé chloroaromatique, un catalyseur à base de palladium et d'une phosphine présentant un pKa supérieur ou égal à 6 avec de l'hydrogène.

2. Procédé selon la revendication 1 caractérisé en ce que le dérivé chloroaromatique répond à la formule ArCl dans laquelle Ar représente un radical aromatique mono, polycyclique ou hétérocyclique éventuellement substitué.

3. Procédé selon la revendication 2 caractérisé en ce que Ar représente un radical benzénique, éventuellement substitué par un groupe alkyle, alkoxy, alkylcarbonyle, cycloalkyle, cycloalkoxy, alkylcarbonyloxy, cycloalkylcarbonyloxy, aryle, aryloxy, arylcarbonyloxy, aryloxycarbonyle ; halogeno, halogénoalkyle, halogénoalkoxy, halogénocycloalkyle, halogénocycloalkyloxy, halogénoaryle, halogénoaryloxy, les motifs alkyle ou alkoxy ayant de 1 à 12 atomes de carbone.

4. Procédé selon la revendication 3 caractérisé en ce que le composé ArCl est le chlorobenzène, le chloroanisole, l'ester éthylique de l'acide chlorobenzoïque.

5.. Procédé selon la revendication 1 caractérisé en ce que la phosphine est choisie parmi les phosphines présentant un angle de cône compris entre 160 et 180° C.

6. Procédé selon les revendications 1 et 5 caractérisé en ce que la phosphine est choisie parmi la tricyclohexyl phosphine, la dicyclohexylmonophénylphosphine, la tribenzylphosphine, la triisopropylphosphine, la triéthylphosphine, la tributylphosphine.

7. Procédé selon les revendications 5 et 6 caractérisé en ce que la phosphine est la tricyclohexylphosphine.

8. Procédé selon la revendication 1 caractérisé en ce qu'on ajoute une base choisie parmi les amines tertiaires et les bases minérales en quantité molaire supérieure au dérivé aromatique.

9. Procédé selon la revendication 1 caractérisé en ce que la réaction a lieu dans un excès de réactif ou en présence d'un solvant choisi parmi les dérivés aromatiques ou aliphatiques hydrocarbonés, eventuellement halogénés, les éthers, les alcools, les cétones, les amides, les nitriles.

10. Procédé selon la revendication 1 caractérisé en ce que la quantité de palladium exprimé en milliatome-gramme de métal noble ou en millimoles de dérivé métallique par litre est comprise dans l'intervalle $10^{-5}$ à 100.

11. Procédé selon les revendications 1 et 10 caractérisé en ce que la quantité de phosphine est telle que le nombre de moles de phosphine au nombre des atomes-grammes de métal soit compris entre 2 et 10000 et de préférence entre 2 et 5.

12. Procédé selon la revendication 1 caractérisé en ce que la pression réactionnelle est comprise entre 1 et 300 bar et de préférence entre 10 et 100 bar.

13. Procédé selon la revendication 1 caractérisé en ce que la température réactionnelle est comprise entre 50 et 250° C et de préférence comprise entre 100 et 200° C.

14. Procédé de mise en oeuvre caractérisé en qu'on introduit dans un solvant un complexe du palladium de formula (I) suivante

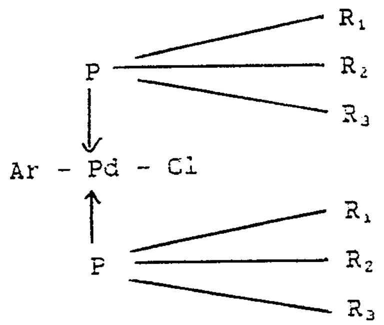

(I)

dans laquelle

. $R_1$, $R_2$, $R_3$ représentent chacun des groupes identiques ou différents choisis parmi les radicaux cyclohexyle, benzyle, isopropyle, l'un des groupes $R_1$, $R_2$ ou $R_3$ pouvant être remplacé par un groupe phényl lorsque les deux autres représentent un groupe cyclohexyle,

. Ar représente un radical aromatique mono, polycyclique ou hétérocyclique avec un dérivé chloroaromatique et de l'hydrogène en présence éventuellement d'un excès de phosphine.

15. Procédé de mise en oeuvre caractérisé en ce qu'on introduit dans un solvant un complexe du palladium de formule (II) suivante

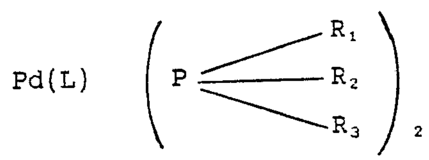

(II)

dans laquelle

. $R_1$, $R_2$, $R_3$ ont la même signification que dans la formule (I) L représente la dibenzylidène acétone ou un groupe alkylène avec un dérivé chloroaromatique et de l'hydrogène en présence éventuellement d'un excès de phosphine.

16. Procédé de mise en oeuvre caractérisé en ce qu'on introduit dans un solvant un complexe de palladium de formule $Pd(L)_3$ dans laquelle L a la même signification que dans la formule (II), un dérivé chloroaromatique et de l'hydrogène en présence d'une phosphine de formule

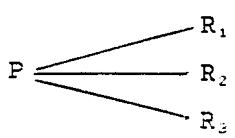

dans laquelle

. $R_1$, $R_2$, $R_3$ ayant la même signification que dans la formule (I).

17. Procédé de mise en oeuvre caractérisé en ce qu'on introduit dans un solvant un complexe de palladium à l'état d'oxydation II, un dérivé chloroaromatique et de l'hydrogène en présence d'une phosphine de formule

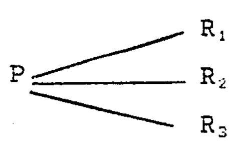

dans laquelle

. $R_1$, $R_2$, $R_3$ ayant la même signification que dans la formule (I).

18. Procédé selon la revendication 17 caractérisé en ce que le complexe de palladium à l'état d'oxydation II est choisi parmi le dichlorure, le dibromure, le diiodure de palladium, le diacétate de palladium, le sulfate de palladium, l'oxyde de palladium.

Office européen des brevets

# RAPPORT DE RECHERCHE EUROPEENNE

Numero de la demande

EP 89 40 1957

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | THE JOURNAL OF ORGANIC CHEMISTRY, vol. 51, no. 5, 7 mars 1986, pages 734-736, American Chemical Society, US; I. PRI-BAR et al.: "Homogeneous, palladium-catalyzed, selective hydrogenolysis of organohalides" * En entier * | 1 | C 07 B 35/06<br>C 07 C 1/26<br>C 07 C 41/24<br>C 07 C 67/30<br>C 07 F 15/00 |
| A | THE JOURNAL OF ORGANIC CHEMISTRY, vol. 42, no. 13, 1977, pages 2309-2313, American Chemical Society, US; H. IMAI et al.: "Transfer hydrogenation and transfer hydrogenolysis. 14. Cleavage of carbon-halogen bond by the hydrogen transfer from organic compounds catalyzed by noble metal salts" * Tableau II, page 2310 * | 1 | |
| A | CHEMICAL ABSTRACTS, vol. 106, no. 11, 16 mars 1987, page 559, résumé no. 84063r, Columbus, Ohio, US; Y. AKITA et al.: "Convenient method for dehalogenation of aryl halides", & SYNTH. COMMUN. 1986, 16(9), 1067-72 * En entier * | 1 | **DOMAINES TECHNIQUES RECHERCHES (Int. Cl.5)**<br>C 07 C 1/00<br>C 07 C 41/00<br>C 07 C 67/00<br>C 07 F 15/00<br>C 07 B 35/00 |

Le présent rapport a été établi pour toutes les revendications

| Lieu de la recherche | Date d'achèvement de la recherche | Examinateur |
|---|---|---|
| LA HAYE | 01-09-1989 | WELLS A.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)